# EUROPÄISCHE PATENTSCHRIFT

(11) **EP 1 191 936 B1**
(45) Veröffentlichungstag und Bekanntmachung des Hinweises auf die Patenterteilung: **07.09.2005**
(21) Anmeldenummer: 01927891.0
(22) Anmeldetag: 06.04.2001
(51) Int. Cl.: A61K 31/70, A23L 1/29, C12P 19/04

(54) **HERSTELLUNG VON POLYGALAKTURONIDEN UND IHRE VERWENDUNG ALS LEBENSMITTELZUSATZSTOFFE**
PRODUCTION OF POLYGALACTURONIDES AND THEIR USE AS FOOD ADDITIVES
PREPARATION DE POLYGALACTURONIDES ET LEUR UTILISATION EN TANT QU'ADDITIFS ALIMENTAIRES

(30) Priorität: 06.04.2000 DE 10019076
(43) Veröffentlichungstag der Anmeldung: 03.04.2002
(73) Patentinhaber: Technische Universität Berlin, 10623 Berlin (DE)
(72) Erfinder: LANG, Christine, 10625 Berlin (DE); DÖRNENBURG, Heike, 10781 Berlin (DE)
(74) Vertreter: Gulde, Klaus W.
(86) Internationale Anmeldenummer: PCT/EP2001/003998
(87) Internationale Veröffentlichungsnummer: WO 2001/076609

(56) Entgegenhaltungen:
- DE-A- 4 223 613
- DE-A- 19 520 743
- US-A- 4 211 799

## Beschreibung

Die Erfindung betrifft Polygalakturonide und deren Verwendung. Polygalakturonide sind Oligosaccharide, welche durch beispielsweise enzymatische Zersetzung von Pektinen erhalten werden. Pektin weist als Hauptbestandteil Galakturonsäure-Monomere bzw. Galakturonsäure(methyl)ester-Monomere auf. Typischerweise sind beide Monomere gleichzeitig anwesend, wobei der Grad der Veresterung der Galakturonsäuregruppen im Bereich 0,5% bis 70% liegt. Die Galakturonsäure- bzw. Ester-Monomere sind α-1,4 miteinander verknüpft. In bestimmten Zonen des Pektinmoleküls sind jedoch Rhamnose-Monomere in die Kette eingefügt mit der Folge, dass dort eine zigzag-Struktur des Polymers entsteht. An den Rhamnose-Monomeren können Seitenketten gebunden sein, welche beispielsweise aus Arabanen (Verknüpfung: α-1,5) und Arabangalaktanen (Verknüpfung: β-1, 3-1, 6-D) gebildet sein können. Die Seitenketten können auch andere Zucker-Monomere enthalten. Ergänzend wird auf die Literaturstelle "Flüssiges Obst", 6/97, Seiten 301 ff., verwiesen. In dieser Literaturstelle ist auch beschrieben, dass bei der Saftherstellung der Einsatz von Pektinasen zum Zwecke der Klärung zu unerwünschten Kolloiden führt, welche die Weiterverarbeitung stören und entfernt oder verhindert werden müssen. Andererseits, in naturtrüben Säften sind nur die Pektine und nicht deren Fragmente auf vorteilhafte Weise viskositätsbeeinflussend. Aus diesen Erwägungen ergibt sich, dass beispielsweise bei der Saftherstellung die Anwesenheit von Pektinfragmenten aus technischen Gründen unerwünscht ist.

Neben technischen Aspekten kommen Pektinen auch physiologische Aspekte zu. Die Literaturstelle Cerda, J.J.; Trans. Am. Clin. Climatol. Assoc., 99:203-213 (1987), beschreibt, dass Pektin aus Grapefruits eine wichtige Rolle in der Gesundheitsförderung bei Konsumenten spielt. Die Literaturstelle Matsumoto, T., et al.; Int. J. Immunopharmacol., 15:683-693 (1993) beschreibt, dass ganz bestimmte Fragmente von Bupleuran 2IIc, einem pektinartigen Polysaccharid aus den Wurzeln von Bupleurum falcatum, von hoher pharmazeutischer Bedeutung sein könnten. Die Fragmente wurden durch Umsetzung mit endo Polygalakturonase erhalten. Schließlich ist es aus der Literaturstelle Voragen, A.G.J., Trends Food Sci. Technol., 9:328-335 (1998), bekannt, dass nicht verdaubare Polysaccharide bzw. Oligosaccharide eine Reihe von gesundheitlich förderlichen Effekten auf eine konsumierende Person haben können.

Aus der Literaturstelle US-A-5,683,991 ist es bekannt, Oligosaccharide mittels Pektinasen aus Pektinen zu pharmazeutischen Zwecken herzustellen. Die dabei eingesetzten Pektinasen sind Mischungen verschiedener Enzyme, wobei möglicherweise auch endo Polygalakturonase enthalten ist, und schneiden dabei aber auch in Bereichen der Seitenketten. Zudem werden die Estergruppen vor der Umsetzung mit den Pektinasen hydrolysiert mit der Folge, dass vergleichsweise kleine Oligosaccharide (2-4 Monomere) erhalten werden.

Zusatzstoffen in der Lebensmittelindustrie kommen die verschiedensten Aufgaben zu. Wesentliche Funktionen liegen in den Bereichen der Herrichtungen von verarbeiteten Lebensmitteln, beispielsweise der Konsistenz, der Haltbarkeit und der farblichen Erscheinung.

Der Erfindung liegt das technische Problem zugrunde, einen Zusatzstoff für Lebensmittel zu schaffen, welcher in gesundheitsfördernder Hinsicht, in geschmacklicher Hinsicht und ggf. in Bezug auf die Konsistenz und/oder andere konsumentenbezogenen Eigenschaften die Lebensmittel verbessert.

Zur Lösung dieses technischen Problems lehrt die Erfindung die Verwendung von Polygalakturoniden als Zusatzstoff in Lebensmitteln, wobei die Polygalakturonide erhältlich sind durch die folgenden Verfahrensschritte:
a) pektinhaltiges Pflanzenmaterial wird einer Pektinextraktion in wäßriger Lösung unterworfen,
b) aus der in Stufe a) erhaltenen Suspension aus Flüssigphase mit gelöstem Pektin und Feststoffen des Pflanzenmaterials werden die Feststoffe abgetrennt,
c) aus der in Stufe b) erhaltenen Flüssigphase wird Pektin ausgefällt,
d) das in Stufe c) erhaltene Pektin wird in eine wäßrige Lösung gebracht und mit gereinigter endo Polygalakturonase geschnitten,
e) die in Stufe d) erhaltenen Poygalakturonide werden ohne weiteren Trennungsschritt und ohne Hydrolyse vorhandener Estergruppen zu einem Polygalakturonid-Präparat aufbereitet.

Wesentlich im Rahmen der Erfindung ist, dass keine Hydrolyse von Estergruppen stattfindet, und dass aufgrund des Einsatzes gereinigter endo Polygalakturonase nur Bindungen bei (natürlicherweise) nicht veresterten Galakturonsäure Monomereinheiten geschnitten werden. Hierdurch wird ein größerer Anteil vergleichsweise großer Oligosaccharide, beispielsweise mit 5 bis 20 Monomereinheiten (Haupt- und ggf. Seitenketten) erhalten. Die Polygalakturonide sind dabei praktisch alle gesättigte Polygalakturonide, sind weniger reaktiv als die durch Pektinlyase- bzw. Pektatlyase-Aktivität entstehenden ungesättigten Polygalakturonide und tragen somit nicht zur nicht-enzymatischen Bräunung und der Entstehung von Hydroxymethylfufural (HMF) aus Fruktose oder Glukose bei. Die so gewonnene Mischung aus verschiedenen Oligosaccharidstrukturen weist besondere Vorteile auf. Einerseits haben die Fragmente mit weitgehend erhaltenen Seitenketten in physiologischer Hinsicht eine das Immunsystem stärkende Wirkung. Die vergleichsweise langen Oligosaccharide der Erfindung wirken zudem als Ballaststoffe, wie andere längerkettige Polymere ebenfalls. Ballaststoffe sind in der Prophylaxe und Therapie vieler Erkrankungen von Bedeutung, beispielsweise Obstipation, Divertikulose, Coloncarcinom, Diabetes mellitus, Lipidstoffwechselerkrankungen. Die Nachteile herkömmlicher Ballaststoffe, nämlich Bindung essentieller Nährstoffe, werden jedoch mit den erfindungsgemäßen Oligosacchariden des Bereiches mit 5 bis 20 Monomereinheiten reduziert. Die erfindungsgemäßen Polygalakturonide lassen sich zudem relativ leicht durch in einem Organismus vorkommende baktarielle Darmflora in kurzkettige Fettsäuren, wie beispielsweise Acetat, Butyrat und Propionat, umwandeln, welche sich wiederum positiv auf die Darmflora und den intestinalen pH-Wert auswirken. Solche Fettsäuren lassen sich vom Organismus energetisch verwerten (Energiegehalt der zugrundeliegenden Oligosaccharide: ca. 2 kcal/g) und dienen insbesondere den Mucosazellen als Energielieferant mit der Folge der Proliferationsmodulation. Die Colonmotilität wird aktiviert und damit die Durchblutung der Mucosa. Die erfindungsgemäß eingesetzten Polygalakturonide sind zudem antibakteriell und emulsionsstabilisierend, was in lebensmitteltechnischer Hinsicht besonders vorteilhaft ist. Letzteres erlaubt den Einsatz auch als Fettersatzstoffe, beispielsweise in Mayonaisen und dergleichen.

Die erfindungsgemäße Verwendung kann mit den verschiedensten aufbereiteten Lebensmitteln erfolgen. Beispiele hierfür sind Fertiggerichte, Babynahrung, Dosenlebensmittel (einschließlich in Glas abgefüllte Lebensmittel), wie Dosengemüse und Dosenfrüchte, Getränke, Süßwaren und Gebäck (einschließlich Chips und dergleichen). Als pektinhaltiges Pflanzenmaterial kommen neben den Pflanzen selbst pflanzliche Extraktionsrückstände, beispielsweise aus der Saftherstellung, sowie pflanzliche Zellkulturen in Frage.

Als als Pektinquelle einzusetzende geeignete Pflanzen sind beispielsweise zu nennen:

Obstarten, insbesondere Apfel und Zitrusfrüchte, Gemüse, insbesondere Zuckerrüben, Karotten und Tomaten. Die Pektinextraktion kann im neutralen Bereich (pH 6,0 - 8,0) oder im Sauren (pH 2,0 - 3,0; Säure: z.B. Schwefelsäure, Salzsäure, Phosphorsäure, Zitronensäure, Milchsäure und/oder Weinsäure) durchgeführt werden. Typische Prozeßbedingungen sind:
40 - 120°C, vorzugsweise 90 - 100°C,
1 - 20 h, vorzugsweise 6 - 10 h, ggf. mehrfache Wiederholung, beispielsweise 2-fach.

Das Abtrennen der Feststoffe kann durch (z.B. hydraulisches) Pressen und/oder Zentrifugation erfolgen. Die danach erhaltene Lösung kann vor der Weiterverarbeitung mittels Vakuumverdampfung und/oder Ultrafiltration konzentriert werden.

Das Ausfällen des Pektins kann auf die verschiedensten Weisen erfolgen. Einerseits können mit Wasser mischbare organische (nichtionische) Lösungsmittel eingesetzt werden, mittels welcher dem Pektin seine Hydrathülle entzogen wird mit der Folge des Ausfallens. In Frage kommen beispielsweise Aceton und C1-C10 Alkylalkohole, wobei aus lebensmitteltechnischer Sicht der Einsatz von Ethanol bevorzugt ist. Alternativ oder zusätzlich können anorganische Salze, beispielsweise die Sulfate und/oder Phosphate des Aluminiums, des Kupfers und/oder des Calciums eingesetzt werden.

Vorzugsweise wird in Stufe d) mit einem pH von 1,4 bis 8,2, vorzugsweise von 3,5 bis 5,0, gearbeitet. Die verwendete endo Polygalakturonase kann aus Pflanzen bzw. darin vorkommenden Mikroorganismen, beispielsweise Baumwolle/Aspergillus flavus/Aspergillus parasiticus, Roggen/Claviceps purpurea, Mais/Cochliobolus carbonum/Fusarium monilifonne, amerikanische Wallnuß/Cryphonectria parasitica, Tomate/Fusarium oxysporum/Ralstonia (Pseudomonas) solanacearum, Reis/Rhizoctonia solani, Gras/Sclerotinia borealis, Sonnenblumen/Sclerotinia sclerotiorum und Apfel/Stereum purpureum, Karotte/Erwinia carotorova, oder Burkholderia (Pseudomonas) cepacia oder aus gentechnisch zur Produktion einer definierten endo Polygalakturonase veränderten Mikroorganismen gewonnen sein. Letzteres ist aufgrund der vergleichsweise einfachen Aufreinigung bevorzugt. Für rekombinantes Arbeiten kommen neben den bekannten Genen der beispielhaft genannten Mikroorganismen auch bekannte cDNAs bzw. cDNAs, welche für bekannte Enzymstrukturen kodieren, von Pflanzen selbst in Frage. Beispiele für bekannte Pflanzen cDNAs sind solche aus Arabidopsis thaliana, Persea americanus und Prunus persica. Beispiele für bekannte endo Polygalakturonasen der Pflanzen sind solche aus Lycopersicon esculentum, Musa acuminata, Gossypium barbadense, Gossypium hirsutum, Cucumus sativus, Phaseolus vulgaris, Citrus limon, Mangifers indica, Cucumis melo, Passiflora edulis, Prunus persica, Pyrus communis, Rubus idaeus und Fragaria ananassa. Bevorzugt wird eine oder mehrere endo Polygalakturonasen ausgewählt aus den endo Polygalakturonasen erhältlich aus den Organismen der Gruppe bestehend aus "Aspergillus carbonarius, Aspergillus niger, Aspergillus oryzae, Aspergillus tubingensis, Aspergillus ustus, Kluyveromyces marxianus, Neurospora crassa, Penicillium frequentans und Saccharomyces cerevisiae SCPP, Lactobacillus casei, Lactobacillus plantarum, Lactococcus lactis, Bacteroides thetaiotaomicron, Piromonas communis, Neocalimastix patriciarum" oder aus mit den endo Polygalakturonasen codierenden DNA Sequenzen dieser Organismen modifizierte andere Mikroorganismen eingesetzt. Die Aufreinigung kann beispielsweise mittels der dem Fachmann gut geläufigen Gelfiltrations-Technologie erfolgen. Die in Stufe d) eingesetzte Menge an Polygalakturonase liegt vorteilhafterweise im Bereich von 10 bis 1000, vorzugsweise im Bereich von 20 bis 400, U/g Pektin. Die Bestimmung der Aktivität erfolgt mit folgender Methode: 10 mg/l Polygalakturonsäure (z.B. Sigma P-7276, gemäß am 01.03.2000 geltenden Produktspezifikationen) werden in Substratpuffer (2 mM Citronensäurelösung, 1 mM CaCl₂) gelöst und mit einer definierten Menge an Enzymlösung versetzt. Inkubation erfolgt für 15 min. bei 25° C. Daraufhin wird die Reaktion gestoppt durch Zugabe eines gleichen Volumens an 4,4 mM 2-Hydroxy-3,5-dinitrobenzoesäurelösung, 5 minütiges Kochen und Abkühlung auf 0° C. Schließlich wird die Absorption bei 540 nm bestimmt und in üblicher Weise gegen eine Standardkurve aus den Werten der Umsatz ermittelt. 1 Unit ist Umsatz von 1µM Galakturonsäure pro Minute.

In einer besonders bevorzugten Ausführungsform der Erfindung ist die Polygalakturonase immobilisiert durch Einschluß in Enzym-Membranreaktoren, bspw. Flachmembran- oder Hohlfasermembranreaktoren, oder Bindung an ein auf übliche Weise hergerichtetes Trägermaterial kann adsorptiv, ionisch, komplexierend, kovalent oder durch Quervernetzung erfolgen.

Die Immobiliesirung erlaubt es, sicherzustellen, dass das Endprodukt praktisch frei von Polygalakturonase ist, und zwar ohne gesonderte Abtrennung. Dadurch ist eine störende Reaktion aufgrund einer ansonsten möglichen Verschleppung eingesetzter Polygalakturonase mit eventuell in dem Lebensmittel enthaltenen Pektinen praktisch ausgeschlossen. Dies ist insbesondere auch deshalb von Vorteil, weil eine unerwünschte Zersetzung von in den Lebensmitteln enthaltenen Pektinen, die Konsistenz der Lebensmittel, beispielsweise deren Viskosität, unvorteilhaft beeinflussen könnte. Zudem ist der Verbrauch an Polygalakturonase vergleichsweise sehr gering.

Die Umsetzung mit Polygalakturonase in Stufe d) erfolgt bevorzugterweise bei 4 bis 80°C, vorzugsweise 30 bis 70° C, und für 2 bis 300 min., vorzugsweise 45 bis 150 min. Dabei kann wahlweise kontinuierlich oder diskontinuierlich (Batchbetrieb) gearbeitet werden. Im Falle des kontinuierlichen Verfahrens bezieht sich die vorstehende Dauer auf eine mittlere Verweilzeit in einem Polygalakturonase enthaltenden Reaktionsraum.

Die erfindungsgemäß hergestellten Polygalakturonide werden typischerweise in Konzentrationen von 0,01 bis 1,0 g/kg Lebensmittel, vorzugsweise von 0,1 bis 0,5 g/kg Lebensmittel, eingesetzt. Dabei sollte die Konzentration so in Abstimmung auf einen üblichen Lebensmittelverbrauch durch einen Menschen eingestellt sein, dass Dosen von 0,5 bis 50 mg, vorzugsweise von 1 bis 25 mg je kg Körpergewicht und je Tag eingehalten werden.

Die Erfindung wird nachfolgend anhand von lediglich Ausführungsbeispielen darstellenden Beispielen näher erläutert.

### Beispiel 1:

### Isolierung und Reinigung einer Polygalakturonase aus Tomaten.

1 kg Tomaten werden in 1 l Wasser homogenisiert und die erhaltene Suspension auf pH 3,0 eingestellt.
Feststoffe (Zellreste) werden durch Zentrifugation (10000 g, 20 min.) abgetrennt und in Wasser gewaschen. Die Pellets werden in 50 mM Natriumacetat/1,25 mM NaCl (pH: 6,0) bei 4° C aufgenommen und bei 4° C für 1 h gerührt. Proteine werden durch 70%ige Ammoniumsulfat-Sättigung ausgefällt und durch Zentrifugieren abgetrennt (10000 g, 20 min.). Das Protein Pellet wird in 0,125 M Natriumacetat (pH: 6,0) gelöst und gegen diesen Puffer dialysiert. Die Proteine werden dann über eine CM-Sepharose-Säule in einem 2-Stufen-Gradienten (0,45 M Natriumacetat, pH 6,0, und 1,0 M Natriumacetat, pH 6,0), getrennt. Die endo Polygalakturonase eluiert mit der ersten Stufe.

### Beispiel 2:

### Herstellung und Reinigung einer Polygalakturonase aus gentechnisch veränderten Mikroorganismen.

Hefe (Saccharomyces cerevisiae) wird mit einem Expressionsplasmid mit cDNA des Aspergillus niger endo Polygalakturonase Gens unter Kontrolle des Hefe-ADH1-Promotors transformiert.
Das Plasmid enthält weiterhin den Hefe-Replikationsursprung und Hefe-Selektionsmarker (z.B. LEU2 Gen). Der erhaltene Hefe-Stamm wird in Nährmedium (Minimalmedium) angezogen und in einem Fermentationsprozess kultiviert. Die endo Polygalakturonase wird dabei in das Nährmedium ausgeschieden.

Die Hefezellen werden aus 500 ml Medium geerntet (Zentrifugieren: 6000 g, 10 min.).

Der klare Mediumüberstand wird auf eine Carboxymethylcellulose Kationenaustauschsäule gegeben und mit 10 mM Natriumacetat (pH 4,0) äquilibriert. Die Proteine werden mit einem linearen Gradienten von 1 bis 1,5 M NaC1 in 10 mM Natriumacetat (pH 4,0) eluiert. Fraktionen mit reiner endo Polygalakturonase eluieren zwischen 0,6 und 0,75 M NaCl.

### Beispiel 3:

### Isolierung und Reinigung einer Polygalakturonase aus einer käuflichen Pektinase.

50 g eines käuflichen Enzymgemisches wird gelöst in 200 ml 0,02 M Natriumacetat (pH 3,6) unter Rühren für 3 Stunden. Feste Bestandteile werden abgetrennt durch Zentrifugieren (25000 g, 10 min.). Die Lösung wird dann mittels einer Sephadex G-50 Säule entsalzt. Die Proteine werden auf eine Alginat-Säule (Matrix durch Crosslinking von Na-Alginat mit Epichlorohydrin) gegeben, und äquilibriert mit 0,02 M Natriumacetat (pH 3,6). Die Elution erfolgt mit 0,1 M Natriumacetat, pH 4,2, dann pH 5,6, und dann mit linearem NaC1-Gradienten von 0 - 1 M in Acetat-Puffer, pH 5,6.

Die Elution der endo Polygalakturonase erfolgt im Salzgradienten.

### Beispiel 4:

### Immobilisierung einer Polygalakturonase.

3 g partikulärer Träger, beispielsweise Silikat- oder Glasträger, welcher mit oberflächengebundenen NH₂-Gruppen (Solvay Enzymes, Hannover) derivatisiert ist, wird in 20 ml 0,01 M Sörensen-Phosphatpuffer (pH 7,0) suspendiert und entgast. 0,2 g bzw. 1000 U endo Polygalakturonase aus Beispiel 3 werden in 5 ml Puffer, pH 7,0, gelöst und zur Trägersuspension gegeben. Unter Rühren wird die Abnahme der Extinktion bei 280 nm über ca. 30 - 60 min. verfolgt. Die Lösung wird abgezogen, es wird 5-fach mit Wasser gewaschen und durch Zugabe von 10 ml GlutardialdehydLösung (5%ig) und 30-minütigem Rühren gekoppelt. Danach erfolgt eine 5-fache Waschung in Wasser, 1 h, gefolgt von 3-facher Waschung mit Puffer (pH 5,0). Schließlich wird in 50 ml Puffer, pH 5,0, suspendiert. Man erhält kovalent immobilisierte endo Polygalakturonase.

### Beispiel 5:

### Herstellung von Oligogalakturoniden aus sichelblättrigem Hasenohr.

1 kg gewaschene Wurzeln des sichelblättrigen Hasenohrs (Bupleurum falcatum, Umbelliferae) werden mechanisch zerkleinert und mit 30 kg deionisiertem Wasser einer Extraktionsverfahrensstufe unterworfen. Die Extraktion wird 2-fach über 8 h bei 98° C und Atmosphärendruck durchgeführt. Nach der Abkühlung auf unter 60° C wird die erhaltene Suspension zur Abtrennung der Feststoffe zentrifugiert (4000 g für 10 min.). Der erhaltene klare Überstand wird mittels Vakuumverdampfung auf 1/10 des ursprünglichen Volumens konzentriert.

Durch Zugabe von 4 Gew.-Teilen Ethanol (96%ig) je Gewichtsteil Lösung wird das Pektin ausgefällt und durch Zentrifugieren (10000 g für 10 min.) bei 4° C, optional mit folgender Dialyse, von der Flüssigphase abgetrennt. Das erhaltene Pektin wird sodann in einem 0,1 M Natriumacetat Puffer (pH: 4,2) gelöst und mit 300 U/g eingesetztem Pektin an endo Polygalakturonase (aus Aspergillus japonicus, erhältlich von der Firma Sigma unter der Produktbezeichnung P 3304, am 01.03.2000 geltende Produktspezifikationen) für 4 h bei 37° C umgesetzt. Die Umsetzung erfolgt dabei batchweise. Die erhaltenen Polygalakturonide werden dann durch Verdampfen des Wassers in fester Form erhalten.

### Beispiel 6:

### Herstellung von Galakturoniden aus Rübenpresschnitzeln.

1 kg Rübenpresschnitzel aus der rübenverarbeitenden Industrie werden in 15 kg saurer wäßriger Lösung (Phosphorsäure, pH: 1,5) für 1 h bei 90° C extrahiert. Nach dem Abkühlen auf 20° C wird die Festphase durch hydraulisches Pressen und ggf. Filtration abgetrennt. Durch Vakuumverdampfung wird eine Konzentration auf 1/10 des Volumens der Flüssigphase durchgeführt. Durch Zugabe von 2 Gew.-Teilen Isopropanol je Gew.-Teil Konzentrat wird das Pektin ausgefällt. Nach Zentrifugation (10000 g, 10 min.) und Trocknung (70° C, 1 h) erfolgt die enzymatische Hydrolyse dadurch, dass zu einer wäßrigen Lösung von 5 g/kg Wasser (eingestellt auf pH 4,0) an Pektinextrakt 20 U/g Pektin an endo Polygalakturonase aus Beispiel 1 zugegeben und die Reaktion für 60 min. bei 60° C durchgeführt wird. Die erhaltenen Polygalakturonide werden dann durch Verdampfen des Wassers in fester Form gewonnen.

### Beispiel 7:

### Herstellung einer Polygalakturonide enthaltenden Babynahrung.

Zu einer als Brei aufbereiteten fertigen Babynahrung werden 0,1 g/kg Brei an Polygalakturoniden aus Beispiel 6 homogen untergemischt und anschließend wird der Brei verkaufsfertig verpackt. Der Brei erweist sich als emulsionsstabilisiert.

### Beispiel 8:

### Herstellung eines Polygalakturonide enthaltenden Erfrischungsgetränkes.

Auf übliche Weise wird aus grünen Teeblättern ein Tee hergestellt. Nach dem Abkühlen werden dem Tee 1 g/kg Getränk an Polygalakturoniden aus Beispiel 5 zugegeben und in Lösung gebracht. Das so erhaltene abfüllungsfertige Produkt weist einen angenehm säuerlichen Geschmack auf.

### Beispiel 9:

### Immobilisierung durch Quervernetzung.

500 mg endo Polygalakturonase werden in 15 ml destilliertem Wasser gelöst. Unter Rühren im Eisbad werden langsam 30 ml eiskaltes Aceton und anschließend 2 ml 25%ige Glutardialdehydlösung zugesetzt. Dann wird 60 min. bei 30°C geschüttelt und anschließend zentrifugiert. Der Überstand wird verworfen und der Rückstand mit 40 ml destilliertem Wasser aufgerührt und mit einem Ultra Turrax homogenisiert. Nach Zentrifugation und Verwerfen des Überstandes wird der Rückstand nochmals mit 40 ml destilliertem Wasser gewaschen. Das erhaltene quervernetzte Präparat wird auf 100 ml suspendiert.

### Beispiel 10:

### Fraktionierung von Polygalakturoniden.

1,5 g der Polygalakturonide aus Beispiel 6 werden in 15 ml destilliertem Wasser in Lösung gebracht. Die Auftrennung der Polygalakturonide erfolgt mittels Chromatographie. Die Lösung wird auf eine mit dem Eluenten (0,2 M Natriumformiat-Puffer, pH 4,7) äquilibrierte Anionenaustauschsäule (2,5 / 40 cm, Bio Rad AGMP 1) mit 150 ml Volumen gegeben. Die Polygalakturonide werden mit einem linearen Gradienten zwischen 0,2 und 0,7 M Natriumformiat-Puffer (pH 4,7) eluiert.

Die Zusammensetzung der individuellen Fraktionen werden sodann mittels Dünnschichtchromatographie (TLC) analysiert. Die Fraktionen werden auf die stationäre Phase, Silicagel 60 (Merck) gegeben; ein Gemisch aus einem Teil Ethanol und einem Teil Acetat (25 mM) wird als mobile Phase verwendet. Die Entwicklung erfolgt bei 35° C. Die individuellen Polygalakturonide werden durch besprühen mit einem Reagenz (200 mg Naphthalene-1,3-diol in 50 ml Methanol und 50 ml 20% (g/g Schwefelsäure) sichtbar. Fraktionen gleicher Zusammensetzung werden zusammengeführt und die in der Fraktion enthaltenen Polygalakturonide mit 2-fachem Volumen an Aceton ausgefällt und nach Zentrifugation (6000 g, 10 min.) vom Aceton getrennt. Man erhält Polygalakturonide definierter Größe.

## Patentansprüche

1. Verwendung von Polygalakturoniden als Lebensmittelzusatzstoffe, wobei die Polygalakturonide erhältlich sind durch die folgenden Verfahrensschritte:
a) pektinhaltiges Pflanzenmaterial wird einer Pektinextraktion in wäßriger Lösung unterworfen,
b) aus der in Stufe a) erhaltenen Suspension aus Flüssigphase mit gelöstem Pektin und Feststoffen des Pflanzenmaterials werden die Feststoffe abgetrennt,
c) aus der in Stufe b) erhaltenen Flüssigphase wird Pektin ausgefällt,
d) das in c) erhaltene Pektin wird in eine wäßrige Lösung gebracht und mit gereinigter endo Polygalakturonase geschnitten,
e) die in Stufe d) erhaltenen Polygalakturonide werden ohne weiteren Trennungsschritt und ohne Hydrolyse vorhandener Estergruppen zu einem Polygalakturonid-Präparat aufbereiten.

2. Verwendung nach Anspruch 1,
wobei das Ausfällen des Pektins mittels Zugabe von C1-C10 Alkylalkoholen und/oder von anorganischen Salzen durchgeführt wird.

3. Verwendung nach Anspruch 1 oder 2,
wobei in Stufe d) ein pH von 1,4 bis 8,2, vorzugsweise von 3,5 bis 5,0, eingestellt wird.

4. Verwendung nach einem der Ansprüche 1 bis 3,
wobei die endo Polygalakturonase mittels der-Gelfiltrations-Technologie gereinigt ist.

5. Verwendung nach einem der Ansprüche 1 bis 4,
wobei in Stufe d) 10 bis 1000, vorzugsweise 20 bis 400, U/g Pektin an Polygalakturonase eingesetzt werden.

6. Verwendung nach einem der Ansprüche 1 bis 5,
wobei die Polygalakturonase immobilisiert ist.

7. Verwendung nach einem der Ansprüche 1 bis 6,
wobei die Umsetzung mit Polygalakturonase in Stufe d) bei 4 bis 80° C, vorzugsweise 30 bis 70° C, und für 2 bis 300 min., vorzugsweise 45 bis 150 min., durchgeführt wird.

## Claims

1. The use of polygalacturonides as food additives, which polygalacturonides are obtainable by means of the following processing steps:
a) subjecting vegetable matter containing pectin to a pectin extraction process in an aqueous solution,
b) separating the solids from the suspension obtained in step a), which suspension comprises a liquid phase containing dissolved pectin and solids stemming from said vegetable matter,
c) precipitating pectin from the liquid phase obtained in step b),
d) dissolving the pectin obtained in step c) in an aqueous medium and cleaving said pectin with purified endopolygalacturonase,
e) processing the polygalacturonides obtained in step d) into a polygalacturonide preparation without any further separation steps and without hydrolyzing any existing ester groups.

2. The use according to Claim 1,
wherein said pectin is precipitated by adding C1-C10 alkyl alcohols and/or inorganic salts.

3. The use according to Claim 1 or 2,
wherein the pH value is adjusted to between 1.4 and 8.2, preferably 3.5 and 5.0, in step d).

4. The use according to any one of Claims 1 to 3,
wherein said endopolygalacturonase is purified by means of the gel filtration technology.

5. The use according to any one of Claims 1 to 4,
wherein the amount of polygalacturonase used in step d) ranges between 10 and 1000, preferably 20 and 400 U/g pectin.

6. The use according to any one of Claims 1 to 5,
wherein said polygalacturonase is immobilized.

7. The use according to any one of Claims 1 to 6,
wherein the reaction with polygalacturonase in step d) is carried out at a temperature ranging between 4 and 80° C, preferably 30 and 70° C, and lasts between 2 and 300 min, preferably 45 and 150 min.

## Revendications

1. Utilisation de polygalacturonides comme additifs alimentaires, lesdites polygalacturonides étant rendues disponibles par les étapes de procédé suivantes :
a) le support végétal contenant de la pectine est soumis à une extraction de pectine en solution aqueuse,
b) les substances solides sont séparées de la suspension obtenue en étape a) consistant en une phase liquide avec pectine dissoute et en substances solides du support végétal,
c) la pectine est décantée de la phase liquide obtenue en étape b),
d) la pectine obtenue en c) est ajoutée à une solution aqueuse et coupée avec de l'endo-polygalacturonase purifiée,
e) les polygalacturonides obtenues en étape d) sont traités en préparation polygalacturonide sans autre étape de séparation et sans hydrolyse des groupes esters présents.

2. Utilisation selon la revendication 1,
où la décantation de pectine est effectuée par ajout d'alcools alkyles C1-C10 et/ou de sels inorganiques.

3. Utilisation selon la revendication 1 ou 2,
où, en phase d), le pH est normalisé entre 1,4 et 8,2, préférentiellement entre 3,5 et 5,0.

4. Utilisation selon l'une des revendications 1 à 3,
où l'endo-polygalacturonase est purifiée par procédé de filtration gel.

5. Utilisation selon l'une des revendications 1 à 4,
où, en phase d), sont utilisées de 10 à 1000 unités, préférentiellement de 20 à 400 unités de polygalacturonase par gramme de pectine.

6. Utilisation selon l'une des revendications 1 à 5,
où la polygalacturonase est immobilisée.

7. Utilisation selon l'une des revendications 1 à 6,
où la transformation avec de la polygalacturonase en étape d) est effectuée entre 4 et 80 °C, préférentiellement entre 30 et 70 °C, et pendant une période de 2 à 300 min, préférentiellement de 45 à 150 min.
